# EUROPEAN PATENT SPECIFICATION

(11) **EP 0 599 776 B1**
(45) Date of publication and mention of the grant of the patent: **06.08.1997**
(21) Application number: 93650036.2
(22) Date of filing: 26.11.1993
(51) Int. Cl.: B65D 81/18, B65D 85/72

(54) **Transportation and storage apparatus**
Transport- und Lagerungseinrichtung
Dispositif de transport et de stockage

(30) Priority: 27.11.1992 IE 922848
(43) Date of publication of application: 01.06.1994
(73) Proprietor: BURNS, PHILP & COMPANY LIMITED, Sydney, New South Wales2000 (AU)
(72) Inventor: Smyth, Patrick A., Castleknock, Dublin 15 (IE); O'Driscoll, Patrick, Dungarvan, County Waterford (IE)
(74) Representative: O'Brien, John Augustine

(56) References cited:
- EP-A- 0 117 457
- DE-A- 2 720 694

## Description

The invention relates to the transportation and storage of viscous liquid materials having limited shelf life such as cream yeast.

Yeast used in the manufacture of bread, fermented cakes and the like is generally available in two different forms.

For large scale production a cream yeast is generally provided in bulk at the bakery site. Cream yeast is delivered by bulk tanker to large in-plant holding tanks and the tanks are integrated into the production and control system of the bakery.

For smaller scale production compressed yeast is generally used. This is yeast which is provided in a dry block form of about 1kg weight. A required amount of yeast is cut from the block and is loaded into a mixer in dry form. Compressed yeast however is not as efficient as cream yeast and in some circumstances there may be certain hygiene problems in the manner in which a user handles the compressed yeast.

This invention is directed toward providing a system for transportation and storage of viscous liquid-like materials having limited shelf life such as cream yeast for use in smaller scale production facilities.

More particularly, the invention relates to an apparatus for handling cream yeast and the like materials comprising a housing having means to facilitate transportation of the apparatus.

DE-A-2720694 describes an apparatus of this type which comprises a storage tank for fluids such as for emulsions used as non-stick agents in bakeries. The tank consists of a plastic container supported in a metal frame trolley on wheels.

The invention is characterised in that the housing comprises a holding vessel having an inlet and an outlet, and control means, the control means including a refrigerator unit and a mixer for maintaining the cream yeast at desired conditions in transportation and in use.

Preferably the refrigerator unit includes a chiller element extending into the holding vessel.

In a particularly preferred arrangement the control means includes a temperature probe for monitoring the temperature of the cream yeast in the vessel and controlling the operation of the refrigeration unit.

In a preferred arrangement the control means includes a mixer for the holding vessel.

In a particularly preferred embodiment of the invention the housing is mounted on a chassis which may be adapted to receive the tines of a forklift truck. In addition, or alternatively, the chassis may be a wheeled chassis.

In one arrangement the vessel includes a vent.

In a preferred embodiment of the invention of vessel includes means for washing the vessel, for example a spray ball through which cleaning water is delivered from a cleaning water inlet.

In a particularly preferred embodiment to the invention the vessel includes an inspection and cleaning hatch.

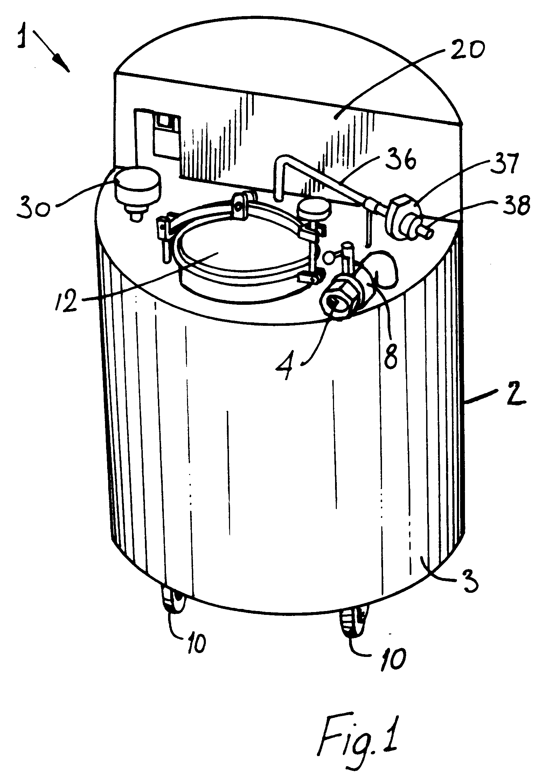

The invention will he more clearly understood from the following description thereof, given by way of example only with reference to the accompanying drawings in which:-
Fig. 1 is a perspective view of an apparatus for handling cream yeast according to the invention;

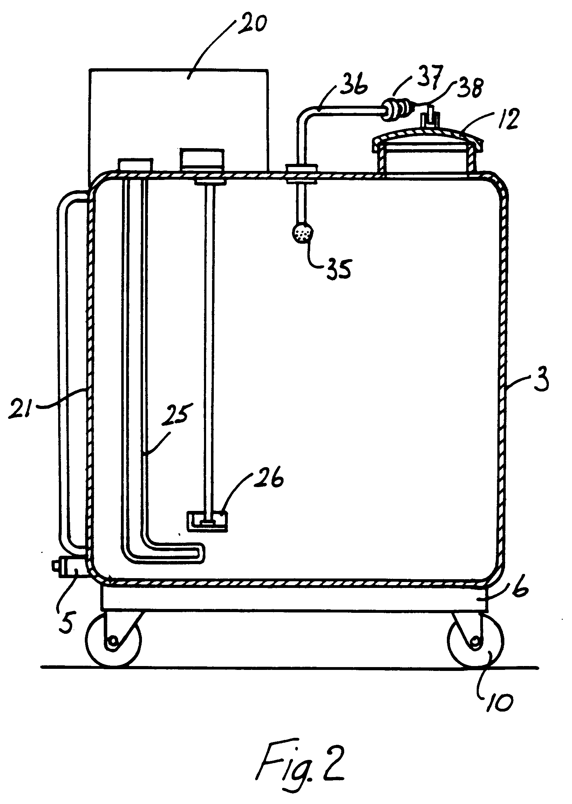

Fig. 2 is a side, partially cross sectional view of the apparatus of Fig. 1;

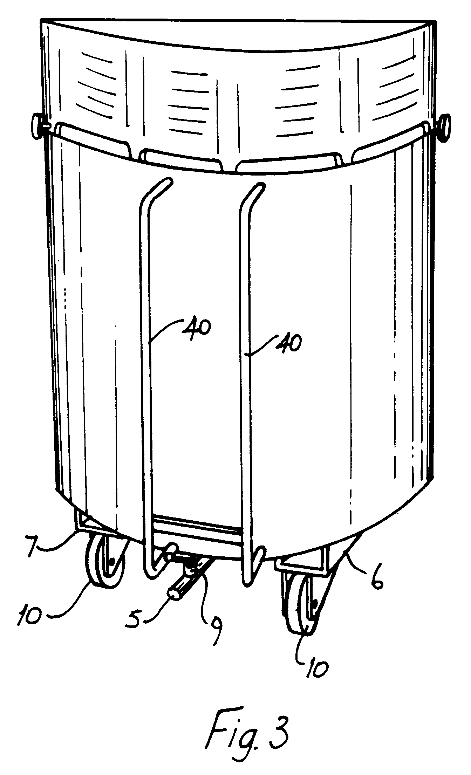

Fig. 3 is a rear view of the apparatus; and

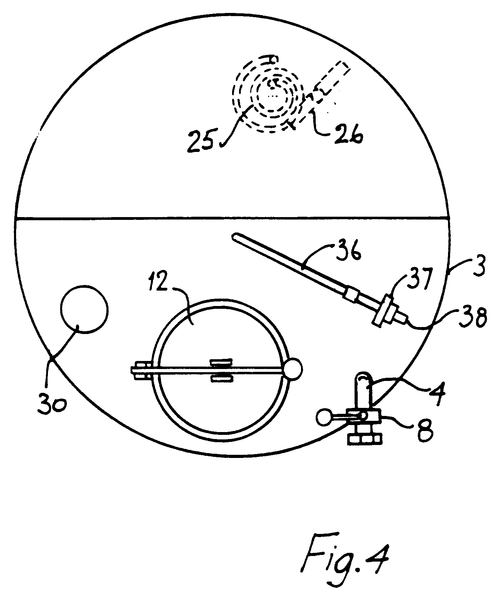

Fig. 4 is a plan view of the apparatus.

Referring to the drawings there is illustrated an apparatus for handling paste-like materials having limited shelf life, in this case for handling cream yeast material, the apparatus being indicated generally by the reference numeral 1. The apparatus 1 comprises a housing 2 incorporating a holding vessel 3 having an inlet spigot 4 and an outlet spigot 5 fitted with valves 8, 9 respectively and control means for maintaining the cream yeast material in the holding vessel 3 at desired conditions in transportation and in use. The housing 2 is mounted on a chassis including a pair of channel members 6, 7 which are sized and shaped to receive the tines of a forklift truck (not shown) used for loading and unloading the apparatus from a truck or the like. Castors or wheels 10 are also provided. Handles 40 facilitate moving the apparatus on site. An access hatch 12 allows for inspection of the contents of the vessel 3 and access for cleaning and maintenance.

Control means for maintaining the cream yeast at desired condition in the vessel 3 includes a temperature control means, in this case a refrigerator unit 20, mounted in the housing 2. A temperature probe 21 on the side wall of the vessel 3 monitors the temperature of the yeast in the vessel 3 and controls the operation of the refrigerator unit 20 to deliver a refrigerant down a chiller coil 25 which extends into the cream yeast in the vessel 3.

The control means also includes a mixer 26 which extends into the vessel 3 to maintain the contents in mixed condition during transportation, storage and use. An automatically operated vent 30 communicates between the vessel 3 and the exterior of housing 2.

Preferably, the power required for operating the refrigerator unit 20 and the mixer 26 is provided along a common supply cable having a plug at one end associated with the housing 2 to which a power supply may be connected during transportation, disconnected on site and connected to a local site power supply so that there is a constant power supply for maintaining the desired conditions of the cream yeast in the vessel 3.

For cleaning the vessel 3 a spray ball 35 is provided inside vessel 3 at the upper end thereof. Cleaning water to the spray ball 35 is provided along a cleaning water inlet line 36 having a valve 37 and a spigot 38 to facilitate connection to an on-site water supply.

In use, cream yeast is loaded into the vessel 3 by connecting a supply line to the inlet spigot 4 and opening the valve 8. Power supply to the refrigerator unit 20 and the mixer 26 is switched on to maintain the cream yeast at desired conditions in the vessel 3. The valve 8 is then closed and the supply line disconnected from the spigot 4. The apparatus may then be lifted onto a truck using a forklift or the like and connected to a power supply from the truck to maintain the desired conditions of the cream yeast during transportation. When the truck arrives at a bakery site a forklift is again used to remove the housing 2 from the truck and, typically using the wheels 10 and handles 40, is moved into a desired position. Generally it will be replacing an empty apparatus of similar type at the bakery site. The empty apparatus is removed and taken back to the yeast supplier for cleaning and refilling. On site, the apparatus with the cream yeast in the vessel 3 is connected up by connecting to a local power supply for the refrigerator unit 20 and mixer 26 and a yeast outlet line is connected to the spigot 5. When the valve 9 is opened the apparatus is ready for operation to deliver desired amounts of cream yeast into a mixer in the bakery.

The apparatus provides a system of transportation, handling and storage of cream yeast material which allows the considerable cost and hygiene advantage of cream yeast materials to a smaller bakery to be attained. When the cream yeast has been emptied from the vessel 3 the power and other supply lines are disconnected and another full apparatus is inserted in its place.

## Claims

1. An apparatus (1) for handling cream yeast and the like materials comprising a housing having means (6,7,10,40) to facilitate transportation of the apparatus (1), characterised in that the housing (2) comprises a holding vessel (3) having an inlet (4) and an outlet (5) and control means (20,21,25,26), the control means (20,21,25,26) including a refrigerator unit (20,25) and a mixer (26) for maintaining the cream yeast at desired conditions in transportation and in use.

2. Apparatus as claimed in Claim 1 wherein the refrigerator unit (20,25) includes a chiller element (25) extending into the holding vessel (3).

3. Apparatus as claimed in Claim 1 or 2 wherein the control means (20,21,25) includes a temperature probe (21) for monitoring the temperature of the cream yeast in the vessel (3) and controlling the operation of the refrigeration unit (20).

4. Apparatus as claimed in any preceding claim wherein the housing (2) is mounted on a chassis (6,7) which is preferably adapted to receive the tines of a forklift truck, typically the chassis is a wheeled chassis.

5. Apparatus as claimed in any preceding claim wherein the vessel (3) includes a vent (30).

6. Apparatus as claimed in any preceding claim wherein the vessel (3) includes means (35) for washing the vessel, preferably the means for washing the vessel comprises a spray ball (35) in the vessel (3) through which cleaning water is delivered from a cleaning water inlet (36).

7. Apparatus as claimed in any preceding claim wherein the vessel (3) includes an inspection and cleaning hatch (12).

## Patentansprüche

1. Ein Apparat (1) zur Behandlung von aufgerahmter Hefe und gleichartigen Materialien, der ein Gehäuse mit Mitteln (6, 7, 10, 40) aufweist, die den Transport des Apparats (1) ermöglichen, dadurch gekennzeichnet, daß das Gehäuse (2) ein Speichergefäß (3) mit einem Einlaß (4) und einem Auslaß (5) und Regelungsmittel (20, 21, 25, 26) aufweist, wobei die Regelungsmittel (20, 21, 25, 26) eine Kälteeinheit (20, 25) und einen Rührer (26) umfassen, um die aufgerahmte Hefe auf dem Transport und während Gebrauch in dem gewünschten Zustand zu halten.

2. Ein Apparat nach Anspruch 1, wobei die Kälteeinheit (20, 25) ein Kühlelement (25) aufweist, das in das Speichergefäß (3) hineinragt.

3. Ein Apparat nach Anspruch 1 oder 2, wobei die Regelungsmittel (20, 21, 25) einen Temperaturfühler (21) umfassen, um die Temperatur der aufgerahmten Hefe in dem Gefäß (3) zu überwachen und um den Betrieb der Kälteeinheit (20) zu regeln.

4. Ein Apparat nach irgendeinem vorstehenden Anspruch, wobei das Gehäuse (2) auf einem Chassis (6, 7) montiert ist, das vorzugsweise zur Aufnahme der Gabeln eines Gabelstaplers adaptiert ist, wobei das Chassis kennzeichnend mit Laufrädern versehen ist.

5. Ein Apparat nach irgendeinem vorstehenden Anspruch, wobei das Gefäß (3) mit einer Entlüftungsöffnung (30) versehen ist.

6. Ein Apparat nach irgendeinem vorstehenden Anspruch, wobei das Gefäß (3) Mittel (35) zum Waschen des Gefässes aufweist, wobei die Mittel zum Waschen des Gefässes vorzugsweise eine Sprühkugel (35) in dem Gefäß (3) aufweisen, durch welche Reinigungswasser von einem Reinigungswasserzufluß (36) zugeführt wird.

7. Ein Apparat nach irgendeinem vorstehenden Anspruch, wobei das Gefäß (3) mit einer Kontroll- und Reinigungsöffnung (12) versehen ist.

## Revendications

1. Appareil (1) pour manipuler la levure en crème et les matériaux similaires comprenant un logement ayant des moyens (6, 7, 10, 40) pour faciliter le transport de l'appareil (1), caractérisé en ce que le logement (2) comprend une cuve de retenue (3) ayant une entrée (4) et une sortie (5) et des moyens de commande (20, 21, 25, 26), les moyens de commande (20, 21, 25, 26) comprenant une unité de réfrigération (20, 25) et un mélangeur (26) afin de maintenir la levure en crème dans les conditions souhaitées pendant le transport et l'utilisation.

2. Appareil selon la revendication 1, dans lequel l'unité de réfrigération (20, 25) comprend un élément de refroidissement (25) s'étendant dans la cuve de retenue (3).

3. Appareil selon la revendication 1 ou 2, dans lequel les moyens de commande (20, 21, 25) comprennent une sonde de température (21) pour surveiller la température de la levure en crème dans la cuve (3) et commander le fonctionnement de l'unité de réfrigération (20).

4. Appareil selon l'une quelconque des revendications précédentes, dans lequel le logement (2) est monté sur un châssis (6, 7) qui est de préférence adapté pour recevoir les branches d'un chariot élévateur à fourche, le châssis étant typiquement un châssis à roues.

5. Appareil selon l'une quelconque des revendications précédentes, dans lequel la cuve (3) comprend un évent (30).

6. Appareil selon l'une quelconque des revendications précédentes, dans lequel la cuve (3) comprend des moyens (35) pour nettoyer la cuve, les moyens pour nettoyer la cuve comprenant de préférence une boule de pulvérisation (35) dans la cuve (3) à travers laquelle de l'eau de nettoyage est distribuée depuis une entrée d'eau de nettoyage (36).

7. Appareil selon l'une quelconque des revendications précédentes, dans lequel la cuve (3) comprend une trappe d'inspection et de nettoyage (12).
